(19) Europäisches Patentamt
European Patent Office
Office européen des brevets

(11) EP 0 758 648 B1

(12) **EUROPÄISCHE PATENTSCHRIFT**

(45) Veröffentlichungstag und Bekanntmachung des Hinweises auf die Patenterteilung:
**15.03.2000 Patentblatt 2000/11**

(51) Int Cl.[7]: **C07D 309/32**, A61K 31/35

(21) Anmeldenummer: **96112427.8**

(22) Anmeldetag: **01.08.1996**

(54) **Substituierte 4H-Pyrane mit einer modulierende Wirkung auf Kaliumkanäle**

Substituted 4H-pyrans with a modulating effect on calcium channels

4H-pyranes substitués modulant le canal à calcium

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **14.08.1995 DE 19529858**

(43) Veröffentlichungstag der Anmeldung:
**19.02.1997 Patentblatt 1997/08**

(73) Patentinhaber: **BAYER AG**
**51368 Leverkusen (DE)**

(72) Erfinder:
- **Urbahns, Klaus, Dr.**
  **42115 Wuppertal (DE)**
- **Heine, Hans-Georg, Dr.**
  **47800 Krefeld (DE)**
- **Junge, Bodo, Dr.**
  **42399 Wuppertal (DE)**
- **Mauler, Frank, Dr.**
  **51491 Overrath (DE)**
- **Glaser, Thomas, Dr.**
  **51491 Overrath (DE)**
- **Wittka, Reilinde, Dr.**
  **51069 Köln (DE)**
- **De Vry, Jean-Marie-Viktor, Dr.**
  **51503 Rösrath (DE)**

(56) Entgegenhaltungen:
**EP-A- 0 088 276**

- **J.ORG.CHEM., Bd. 34, Nr. 10, 1969, Seiten 3169-3174, XP002013999 WOLINSKY ET AL: "substituted gamma-Pyrans"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 0 758 648 B1

## Beschreibung

Die vorliegende Erfindung betrifft substituierte 4H-Pyrane, Verfahren zu ihrer Herstellung und ihre Verwendung als Arzneimittel, insbesondere als cerebral wirksame Mittel.
Aus der Publikation J. Org. Chem. (1969), 34 (10), 3169-74 sind einige substituierte 4H-Pyrane bekannt.
Die vorliegende Erfindung betrifft jetzt substituierte 4H-Pyrane der allgemeinen Formel (I),

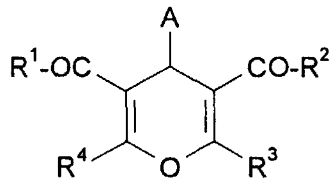

(I)

in welcher

A für Cycloalkyl mit 3 bis 6 Kohlenstoffatomen oder
für geradkettiges oder verzweigtes Alkyl mit bis zu 8 Kohlenstoffatomen steht, oder
für Aryl mit 6 bis 10 Kohlenstoffatomen oder für Pyridyl steht, die gegebenenfalls bis zu 3-fach gleich oder verschieden durch Nitro, Hydroxy, Carboxyl, Cyano, Aryl mit 6 bis 10 Kohlenstoffatomen, Halogen, Cycloalkyl mit 3 bis 6 Kohlenstoffatomen, Trifluormethyl oder durch geradkettiges oder verzweigtes Alkyl, Alkoxycarbonyl, Alkylthio oder Alkoxy mit jeweils bis zu 6 Kohlenstoffatomen oder durch eine Gruppe der Formel -O-CO-$R^5$ substituiert sind,
worin

$R^5$ geradkettiges oder verzweigtes Alkyl mit bis zu 6 Kohlenstoffatomen bedeutet,

$R^1$ und $R^2$ gleich oder verschieden sind und für Wasserstoff, Amino oder für geradkettiges oder verzweigtes Alkyl, Alkoxy oder Alkylamino, mit jeweils bis zu 8 Kohlenstoffatomen stehen,

$R^3$ und $R^4$ gleich oder verschieden sind und für geradkettiges oder verzweigtes Alkyl oder Acyl mit jeweils bis zu 6 Kohlenstoffatomen stehen,

und deren Salze,
wobei 3,5-Diacetyl-2,4,6-trimethyl-4H-Pyran, 3,5-Diethoxycarbonyl-2,6-dimethyl-4-phenyl-4H-Pyran und 3,5-Diethoxycarbonyl-2,4,6-trimethyl-4H-Pyran ausgenommen sind.
Im Rahmen der Erfindung sind physiologisch unbedenkliche Salze bevorzugt. Physiologisch unbedenkliche Salze sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.
Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.
Bevorzugt sind Verbindungen der allgemeinen Formel (I),
in welcher

A für Cyclopropyl, Cyclobutyl, Cyclopentyl oder Cyclohexyl steht, oder für geradkettiges oder verzweigtes Alkyl mit bis zu 6 Kohlenstoffatomen steht, oder
für Phenyl, Naphthyl oder Pyridyl steht, die gegebenenfalls bis zu 3-fach gleich oder verschieden durch Nitro, Cyano, Fluor, Chlor, Brom, Iod, Phenyl, Naphthyl, Trifluormethyl, Hydroxy, Carboxyl, Cyclopropyl, Cyclopentyl, Cyclohexyl oder durch geradkettiges oder verzweigtes Alkyl, Alkoxycarbonyl, Alkylthio oder Alkoxy mit jeweils bis zu 4 Kohlenstoffatomen oder durch eine Gruppe der Formel -O-CO-$R^5$ substituiert sind,

worin

$R^5$ geradkettiges oder verzweigtes Alkyl mit bis zu 4 Kohlenstoffatomen bedeutet,

$R^1$ und $R^2$ gleich oder verschieden sind und für Wasserstoff, Amino oder für geradkettiges oder verzweigtes Alkyl, Alkoxy oder Alkylamino mit jeweils bis zu 6 Kohlenstoffatomen stehen,

$R^3$ und $R^4$ gleich oder verschieden sind und für geradkettiges oder verzweigtes Alkyl oder Acyl mit jeweils bis zu 4 Kohlenstoffatomen stehen,

und deren Salze,
wobei 3,5-Diacetyl-2,4,6-trimethyl-4H-Pyran, 3,5-Diethoxycarbonyl-2,6-dimethyl-4-phenyl-4H-Pyran und 3,5-Diethoxycarbonyl-2,4,6-trimethyl-4H-Pyran ausgenommen sind.

Besonders bevorzugt sind Verbindungen der allgemeinen Formel (I), in welcher

A für Cyclopropyl, Cyclohexyl oder für geradkettiges oder verzweigtes Alkyl mit bis zu 4 Kohlenstoffatomen steht, oder
für Naphthyl oder Phenyl steht, die gegebenenfalls bis zu 3-fach gleich oder verschieden durch Nitro, Cyano, Fluor, Chlor, Brom, Iod, Phenyl, Naphthyl, Hydroxy, Carboxyl, Cyclopropyl, Cyclopentyl, Cyclohexyl, Trifluormethyl oder durch geradkettiges oder verzweigtes Alkyl, Alkoxycarbonyl, Alkylthio oder Alkoxy mit jeweils bis zu 4 Kohlenstoffatomen oder durch eine Gruppe der Formel -O-CO-$R^5$ substituiert sind,
worin

$R^5$ geradkettiges oder verzweigtes Alkyl mit bis zu 4 Kohlenstoffatomen bedeutet,

$R^1$ und $R^2$ gleich oder verschieden sind und für Wasserstoff, Amino oder für geradkettiges oder verzweigtes Alkyl, Alkoxy oder Alkylamino, mit jeweils bis zu 4 Kohlenstoffatomen stehen,

$R^3$ und $R^4$ gleich oder verschieden sind und für geradkettiges oder verzweigtes Alkyl oder Acyl mit jeweils bis zu 4 Kohlenstoffatomen stehen,

und deren Salze,
wobei 3,5-Diacetyl-2,4,6-trimethyl-4H-Pyran, 3,5-Diethoxycarbonyl-2,6-dimethyl-4-phenyl-4H-Pyran und 3,5-Diethoxycarbonyl-2,4,6-trimethyl-4H-Pyran ausgenommen sind.

Außerdem wurde ein Verfahren zur Herstellung der erfindungsgemäßen Verbindungen der allgemeinen Formel (I) gefunden, dadurch gekennzeichnet, daß man

[A] Aldehyde der allgemeinen Formel (II)

$$A\text{-}CHO \qquad (II)$$

in welcher

A die oben angegebene Bedeutung hat,

mit Verbindungen der allgemeinen Formel (III)

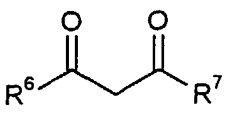

(III)

in welcher

$R^6$ die oben jeweils angegebenen Bedeutungen von $R^1$ und $R^2$ umfaßt,

und

$R^7$ die oben angegebenen Bedeutungen von $R^3$ und $R^4$ umfaßt,

oder

[B] Ylidenverbindungen der allgemeinen Formel (IV)

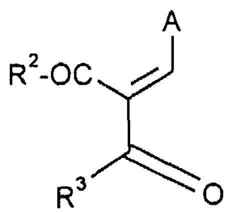

(IV)

in welcher

A, $R^2$ und $R^3$ die oben angegebenen Bedeutungen haben,

mit Verbindungen der allgemeinen Formel (IIIa) oder (IIIb)

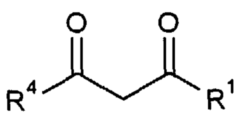

(IIIa)

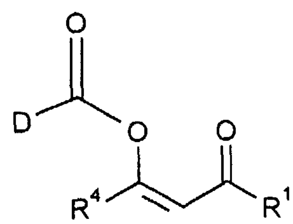

(IIIb)

in welchen

$R^1$ und $R^4$ die oben angegebenen Bedeutungen haben und

D zusammen mit der CO-Gruppe einer elektronenziehenden, aktivierten Rest bildet, wobei D beispielsweise für Wasserstoff, Trifluormethyl, Phenyl oder für $C_1$-$C_4$-Alkyl, vorzugsweise für Methyl steht,

in inerten Lösemitteln in Anwesenheit von Hilfsstoffen und/oder in Anwesenheit eines wasserentziehenden Mittels umsetzt,
und im Fall der Verbindungen der allgemeinen Formel (I), in welcher $R^1$/$R^2$ für Amino und/oder Alkylamino stehen, ausgehend von den Estern zunächst durch Verseifung die entsprechenden Säuren herstellt, durch Umesterung z.B. mit Thionylchlorid in die Carbonsäurechloride überführt und in einem letzten Schritt mit Ammoniak oder Alkylaminen umsetzt.

Die erfindungsgemäßen Verfahren können durch folgendes Formelschema beispielhaft erläutert werden:

1)

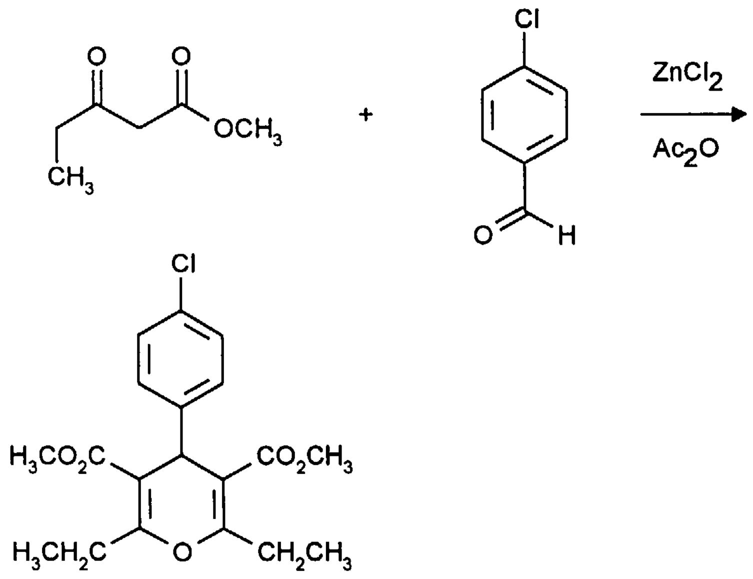


2)

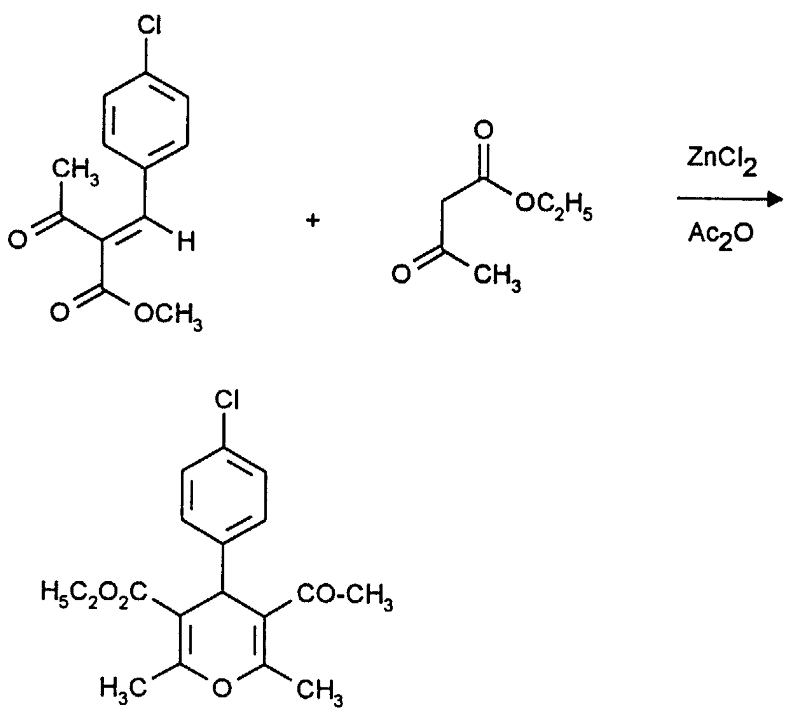

3)

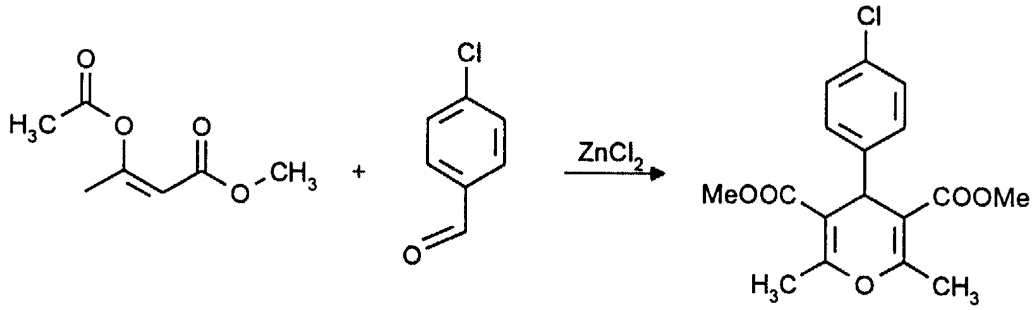


4)

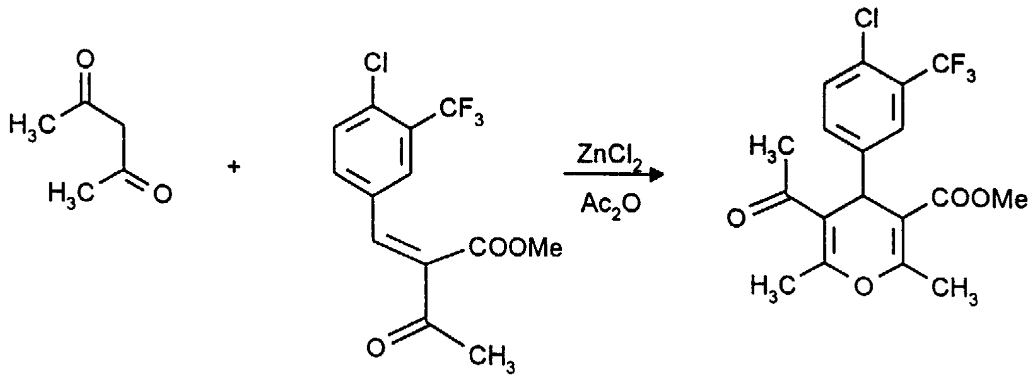


5)

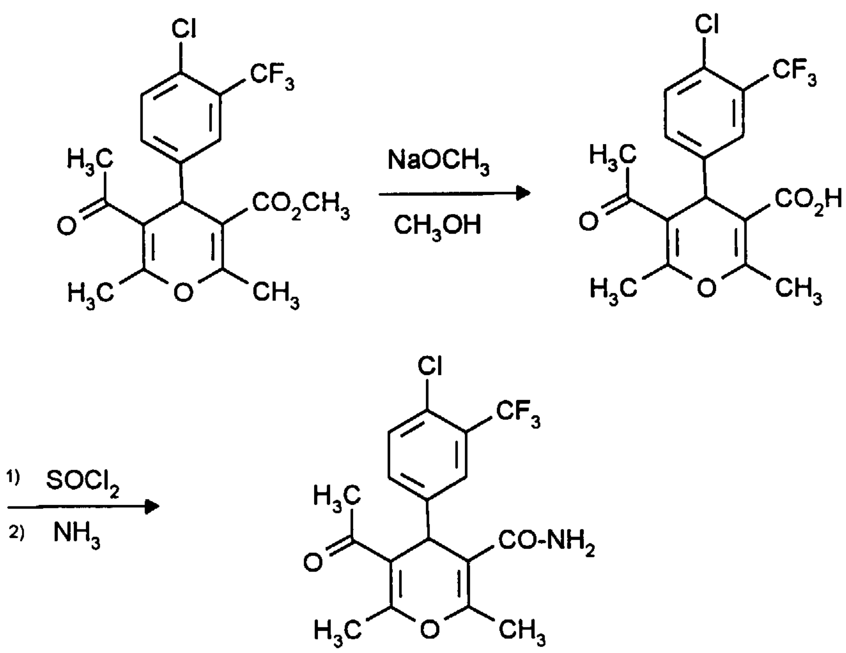


Als Lösemittel eignen sich alle inerten organischen Lösemittel, die sich unter den Reaktionsbedingungen nicht verändern. Hierzu gehören bevorzugt Ether wie Diethylether, Dioxan, Tetrahydrofuran, Glykoldimethylether, oder Diethylenglykoldimethylether, Essigsäureethylester oder Acetonitril, oder Amide wie Hexamethylphosphorsäuretriamid

oder Dimethylformamid, oder halogenierte Kohlenwasserstoffe wie Methylenchlorid, Tetrachlorkohlenstoff oder Kohlenwasserstoffe wie Benzol oder Toluol, oder Pyridin oder Carbonsäuren wie AcOH oder $CF_3COOH$. Ebenso ist es möglich, Gemische der genannten Lösemittel zu verwenden. Bevorzugt ist Eisessig.

Als wasserentziehende Mittel eignen sich Säureanhydride und -chloride wie Essigsäureanhydrid ($Ac_2O$), Essigsäurechlorid (AcCl), $POCl_3$, $SOCl_2$, $SO_2Cl_2$ oder Molekularsiebe. Bevorzugt ist $Ac_2O$. Das Reaktionswasser kann auch azeotrop entfernt werden.

Werden Enolacetate eingerührt, ist kein wasserentziehendes Mittel vonnöten.

Als Hilfsstoffe eignen sich beispielsweise $TiCl_4$, $SnCl_4$, $BF_3$ x $OEt_2$, $Zn(OAc)_2$, $LiClO_4$ oder Zinkchlorid. Bevorzugt ist Zinkchlorid.

Der Hilfsstoff wird in einer Menge von 0,1 mol bis 5 mol, bevorzugt von 1 mol bis 2 mol, jeweils bezogen auf 1 mol der Verbindungen der allgemeinen Formeln (II) und (IV) eingesetzt.

Das Verfahren wird im allgemeinen in einem Temperaturbereich von 0°C bis 150°C, bevorzugt von 40°C bis 80°C durchgeführt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Verbindungen der allgemeinen Formeln (II), (III), (IIIa), (IIIb) und (IV) sind an sich bekannt oder nach üblichen Methoden herstellbar.

Enantiomerenreine Formen erhält man z.B., indem man Diastereomerengemische der Verbindungen der allgemeinen Formel (I), in welcher $R^1$ für einen optisch aktiven Esterrest steht, nach üblicher Methode trennt, anschließend entweder direkt umestert oder zuerst die chiralen Carbonsäuren herstellt und dann durch Veresterung die enantiomerenreinen Verbindungen herstellt.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

Die Erfindung betrifft auch die Verwendung der bekannten Verbindungen 3,5-Diacetyl-2,4,6-trimethyl-4H-pyran,3,5-Diethoxycarbonyl-2,6-dimethyl-4-phenyl-4H-pyran und 3,5-Diethoxycarbonyl-2,4,6-trimethyl-4H-pyran und der erfindungsgemäßen Verbindungen als Arzneimittel, insbesondere als cerebral wirksame Arzneimittel.

Die bekannten Verbindungen und die erfindungsgemäßen Verbindungen der allgemeinen Formel (I) zeigen ein nicht vorhersehbares, wertvolles pharmakologisches Wirkspektrum.

Sie sind Modulatoren mit Selektivität für calciumabhängige und Charybdotoxinsensitive Kalium-Kanäle (IK (Ca)-Kanäle), insbesondere des zentralen Nervensystems. Aufgrund ihrer pharmakologischen Eigenschaften können sie für die Herstellung von Arzneimitteln, insbesondere von Arzneimitteln zur Behandlung von zentral degenerativen Erkrankungen eingesetzt werden, wie bei Auftreten von Demenzen, wie Multiinfarktdemenz (MID), primär degenerativer Demenz (PDD), präseniler und seniler Demenz bei Alzheimerscher Krankheit, HIV-Demenz und andere Demenzformen. Ferner eignen sie sich zur Behandlung von Parkinsonscher Krankheit oder amyotrophischer Lateralsklerose sowie multipler Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe und Behandlung, und zur Bekämpfung der Folgen cerebraler Durchblutungsstörungen wie cerebraler Ischämien, Schlaganfällen, Schädel-Hirn-Traumata und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüber hinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Bluthochdruck, Arrhythmie, Angina, Diabetes, Sichelzellanämie, Krebs, Restenase, chronisch obturierenden Lungenerkrankungen und Ödemen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

**Rubidium-Efflux aus C6-BU1-Glioma-Zellen**

Die Versuche wurden mit geringfügigen Veränderungen entsprechend der von Tas et al. (Neurosci. Lett. 94, 279-284, (1988)) beschriebenen Methode durchgeführt.

Dazu werden Ratten C6-BU1-Glioma-Zellen verwendet. Die Detektion erfolgt über Atom Absorptionsspektroskopie.

**Beispiele**

**Beispiel 1**

4-(4-Chlorphenyl)-2,6-diethyl-4H-pyran-3,5-dicarbonsäuredimethylester

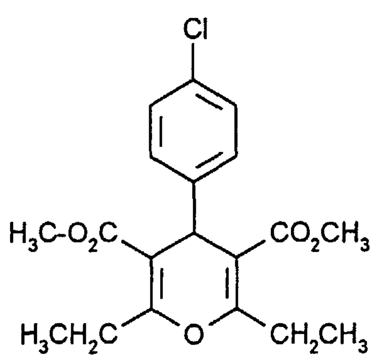

7,0 g (0,05 mol) 4-Chlorbenzaldehyd, 15,6 g (0,12 mol) 3-Oxovaleriansäuremethylester und 6,8 g (0,05 mol) wasserfreies Zinkchlorid werden in dem Gemisch von 9,3 g Eisessig und 10,4 g Acetanhydrid unter Rühren gelöst und anschließend 5 Wochen bei Zimmertemperatur (20-25°C) stehen gelassen. Die homogene Lösung wird in 100 g Eis eingetragen und mit Dichlormethan extrahiert. Die Dichlormethanextrakte werden nacheinander mit Wasser, gesättigter Natriumbicarbonat-Lösung und Wasser gewaschen. Nach Trocknen der Dichlormethanphase mit wasserfreiem Natriumsulfat wird filtriert, das Filtrat i.Vak. eingedampft, und der Rückstand (20,3 g) an 600 g Kieselgel mit Toluol/Essigsäureethylester (10:1) chromatographiert. 2,7 g eines einheitlichen farblosen Öls werden erhalten, das durch Kugelrohrdestillation (200°C/0,01 mbar) von Lösungsmittelresten befreit wird.
$R_f$-Wert (Toluol/Essigsäureethylester = 10:1): 0,52

| | | |
|---|---|---|
| $C_{19}H_{21}ClO_5$ (364,8) | Ber. C: 62,55 %; | H: 5,80 % |
| | Gef. C: 62,20 %; | H: 5,86 % |

$^1$H-NMR ($CDCl_3$): $\delta$ = 7,26-7,13 m (4, aromat. Protonen), 4,73 s (1, C4-H), 3,64 s (6, C3-$COOCH_3$ und C5-$COOCH_3$),

2,84 u. 2,74 dddd, erscheint als 12-Liniensignal (4, C2-$CH_2$- und C6-$CH_2$-) und 1,90 ppm t (6, C2-$CH_2$-$CH_3$ und C6-$CH_2$-$CH_3$).

**Beispiel 2**

3,5-Diacetyl-4-(4-chlorphenyl)-2,6-dimethyl-4H-pyran

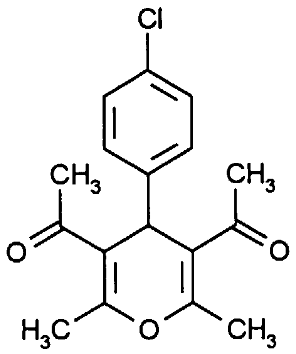

7,0 g (0,05 mol) 4-Chlorbenzaldehyd, 12,0 g ( 0,12 mol) Pentan-2,4-dion und 6,8 g (0,05 mol) wasserfreies Zinkchlorid werden in dem Gemisch aus 8,9 ml Eisessig und 9,6 ml Acetanhydrid 3 Wochen bei Zimmertemperatur stehen gelassen. Der Ansatz wird auf Eis gegeben und mit Dichlormethan extrahiert. Die organischen Extrakte werden nacheinander mit gesättigter Natriumbicarbonat-Lösung und Wasser gewaschen, über wasserfreiem Natriumsulfat geklärt und filtriert. Eindampfen i.Vak. liefert 13,3 g Öl.

Das aus zwei gleichen Ansätzen erhaltene Rohprodukt wird der Blitzchromatographie an Kieselgel mit Toluol/Essigsäureethylester (steigender Gradient) unterworfen und ergibt 9,6 g eines Gemisches aus 2-Acetyl-4-(4-chlorphenyl)-buten-2-on und 3,5-Diacetyl-4-(4-chlorphenyl)-2,6-dimethyl-4H-pyran. Rechromatographie an 7oo g Kieselgel mit Toluol bzw. Toluol/Essigsäureethylester = 20:1, ergibt 4,1 g einheitliches Produkt.
Schmp.: 86-88°C (Kap.) (aus Dichlormethan/Petrolether).
$R_f$-Wert: 0,20 (Toluol/Essigsäureethylester = 10:1).

| | | |
|---|---|---|
| $C_{17} H_{17} Cl O_3$ (304,8) | Ber. C: 67,00 % | H: 5,62% |
| | Gef. C: 66,9% | H: 5,77% |

$^1$H-NMR($CDCl_3$): $\delta$ = 7,27 - 7,15 m (4, aromat. Protonen), 4,88 s (1, C4-H), 2,32 s (6, C3-$COCH_3$ u. C5-$COCH_3$) und 2,21 ppm s (C2-$CH_3$ u. C6-$CH_3$).

**Beispiel 3**

4-(4-Chlorphenyl)-2,6-dimethyl-4H-pyran-dicarbonsäuredimethylester

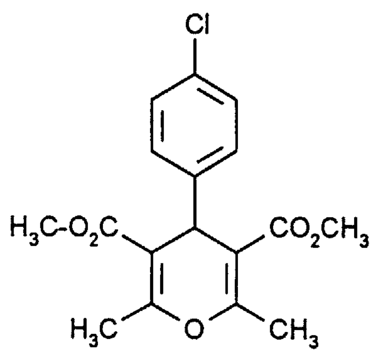

a) In das Gemisch aus 9,6 ml Acetanhydrid und 6,8 g (0,05 mol) wasserfreiem Zinkchlorid werden bei 25°C unter Rühren 13,9 g (0,12 mol) Acetessigsäuremethylester und 7,0 g (0,05 mol) 4-Chlorbenzaldehyd eingetragen. Unter Temperaturanstieg (auf ca. 60°C) wird eine hellgelbe Lösung erhalten, die 3 mal 8h auf 60-65°C erwärmt wird. Zur Aufarbeitung wird das Reaktionsgemisch in 200 ml Eiswasser eingetragen und mit Dichlormethan extrahiert. Die organische Phase wird nacheinander mit Wasser, gesättigter Natriumbicarbonat-Lösung und wiederum Wasser gewaschen, über wasserfreiem Natriumsulfat geklärt, filtriert und i.Vak. eingedampft. Der Rückstand (18,8 g) wird an Kieselgel (1650 g) mit Toluol/Essigsäureethylester = 25:1 chromatographiert und liefert 9,8 g (58%) einheitliches Pyran sowie 1,2 g eines Gemisches, das zu gleichen Teilen Pyran und 2-Acetyl-3(4-chlorphenyl)acrylsäuremethylester enthält. Kristallisieren der Hauptfraktion aus Petrolether /Diethylether liefert farblose Kristalle vom Schmp. 85-86°C.

b) Die Titelverbindung kann analog durch Umsetzung von 17,4 g (0,11 mol) 3-Acetoxycrotonsäuremethylester, 6,8 g Zinkchlorid und 7 g (0,05 mol) 4-Chlorbenzaldehyd herstellt werden. Nach wäßriger Aufarbeitung erhält man 14,9 g Rohprodukt, das durch Kieselgel aufgetrennt wird und 10,5 g einheitliches Pyran liefert.

$R_f$-Wert (Toluol/Essigsäureethylester = 10:1): 0,41

| | | |
|---|---|---|
| $C_{17}$ $H_{17}Cl$ $O_5$ (336,8) | Ber. C: 60,63 % | H 5,09 % |
| | Gef. C: 60,6 % | H 5,20 % |

$^1$H-NMR ($CDCl_3$): $\delta$ = 7,22-7,14 m (4, aromat. Protonen), 4,74 s (1, C4-H), 3,64 s (6, C3-$COOCH_3$ und C5-$COOCH_3$) und 2,36 ppm s (6, C2-$CH_3$ und C6-$CH_3$).

**Beispiel 4**

4-(4-Chlorphenyl)-2,6-dimethyl-4H-pyran-dicarbonsäure-methylester-ethylester

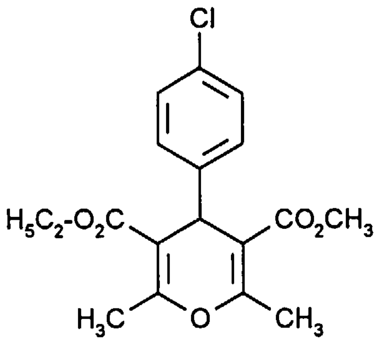

Das Gemisch aus 9,5 g (0,04 mol) 2-Acetyl-3-(4-chlorphenyl)acrylsäuremethylester, 5,7 g (0,044 mol) Acetessigsäureethylester, 5,4 g (0,04 mol) wasserfreiem Zinkchlorid und 7,5 ml Acetanhydrid wird 3x 8h bei 60-65°C gerührt. Das Gemisch wird mit 4,0 ml Eisessig versetzt und weitere 3x 8h bei 60-65°C gerührt. Nach Aufarbeiten, wie voranstehend beschrieben, wird das Rohprodukt (15,9 g) an 2000g Kieselgel mit Toluol chromatographiert. 5,3 g (30 %) kristallines Pyran werden erhalten. Schmp.: 67-69°C (aus Petrolether).
$R_f$-Wert (Toluol/Essigsäureethylester = 10:1): 0,53
$^1$H-NMR($CDCl_3$): $\delta$ = 7,22-7,14 m (4, aromat. Protonen), 4,73 s (1, C4-H), 4,09 2 qu (2, C3-$COOCH_2$-), 3,64 s (3, C5-$COOCH_3$), 2,36 s (6, C2-$CH_3$ und C6-$CH_3$) und 1,20 ppm t (3, C3-$COOCH_2$-$CH_3$).

**Beispiel 5**

5-Acetyl-2,6-dimethyl-4-(4-chlor-3-trifluormethyl)-4H-pyran-3-carbonsäuremethylester

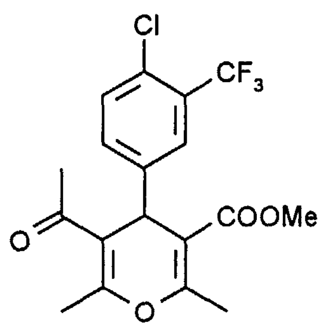

10 g (32,6 mmol) 2-Acetyl-3-(4-chlor-3-trifluormethylphenyl)-acrylsäuremethylester und 10 g (97,8 mmol) 2,4-Pentandion werden mit 8,4 g $ZnCl_2$ in 20 ml Acetanhydrid 3 Stunden bei 60°C gerührt. Nach wäßriger Aufarbeitung und Chromatographie erhält man 2,83 g der Titelverbindung.
Schmelzpunkt: 96°C (Petrolether)
NMR ($CDCl_3$): 2,20 (s,3H), 2,36 (s,6H), 3,69 (s,3H), 4,82 (s,1H), 7,48 (m,2H), 7,51 (s,1H).

| | | | |
|---|---|---|---|
| $C_{18}H_{18}O_4F_3Cl$ (388,77): | Ber.: C: 55,61 %; | H 4,15 %; | O:16,46 % |
| | Gef.: C: 55,55 %; | H 4,17 %; | O:16,32 % |

Zusätzlich fällt Beispiel 5 als Beiprodukt der Synthese von Beispiel 32 an.

In Analogie zu den oben aufgeführten Beispielen 1 - 5 werden die in den Tabellen 1 und 2 aufgeführten Verbindungen hergestellt:

**Tabelle 1:**

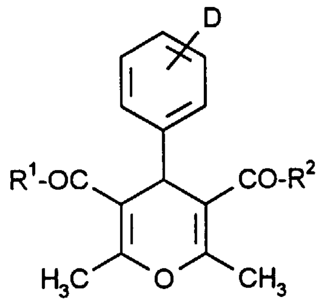

| Bsp.-Nr. | Schmp. (°C) (Kap.) | $R_f$-Wert (LM) | $R^1$ | $R^2$ | D |
|---|---|---|---|---|---|
| 6 | 74-5 | 0,44 (A) | $-OC_2H_5$ | $-OC_2H_5$ | H |
| 7 | 84-5 | 0,44 (A) | $-OC_2H_5$ | $-OC_2H_5$ | $4-OCH_3$ |
| 8 | 89 | 0,45 (A) | $-OC_2H_5$ | $-OC_2H_5$ | $3-NO_2$ |
| 9 | 80 | 0,42 (A) | $-OC_2H_5$ | $-OC_2H_5$ | $2-OCH_3$ |
| 10 | 99 | 0,49 (A) | $-OC_2H_5$ | $-OC_2H_5$ | $4-SCH_3$ |
| 11 | 90 | 0,36 (A) | $-OCH_3$ | $-OCH_3$ | $2-OCH_3$ |
| 12 | 106 | 0,43 (A) | $-OC_2H_5$ | $-OC_2H_5$ | 4-OH |
| 13 | 103-4 | 0,50 (A) | $-OCH_3$ | $-OCH_3$ | $3,4-Cl_2$ |
| 14 | 81-2 | 0,62 (B) | $-OCH_3$ | $-OCH_3$ | $3-CH_3$ |
| 15 | 64-5 | 0,46 (A) | $-OC_2H_5$ | $-OC_2H_5$ | 4-Cl |
| 16 | Oel 200/0,01 mbar | 0,33 (A) | $-OCH_3$ | $-OCH_3$ | $3-COOCH_3$ |
| 17 | 105-9 | 0,42 (A) | $-OCH_3$ | $-OCH_3$ | $4-C_6H_5$ |
| 18 | 122-6 | 0,46 (A) | $-OCH_3$ | $-OCH_3$ | $2-CH_3$ |
| 19 | 80-3 | 0,35 (A) | $-OCH_3$ | $-OCH_3$ | 3-Cl |
| 20 | 62-4 | 0,41(A) | $-OCH_3$ | $-OCH_3$ | H |
| 21 | 73-6 | 0,41 (A) | $-OCH_3$ | $-OCH_3$ | 4-F |
| 22 | 68-9 | 0,47 (A) | $-OCH_3$ | $-OCH_3$ | $3,4-F_2$ |
| 23 | 85-6 | 0,30 (A) | $-OCH_3$ | $-OCH_3$ | $3-OCOCH_3$ |
| 24 | 45-6 | 0,51 (A) | $-O-n-C_3H_7$ | $-O-n-C_3H_7$ | 4-Cl |
| 25 | 51-3 | 0,56 (A) | $-O-n-C_4H_9$ | $-O-n-C_4H_9$ | 4-Cl |
| 26 | 88-9 | 0,46 (A) | $-OC_2H_5$ | $-OC_2H_5$ | $2,3-Cl_2$ |
| 27 | 98-100 | 0,40 (A) | $-OCH_3$ | $OCH_3$ | $2,3-Cl_2$ |
| 28 | 67-9 | 0,53 (A) | $-OCH_3$ | $-OC_2H_5$ | 4-Cl |

| Bsp.-Nr. | Schmp. (°C) (Kap.) | $R_f$-Wert (LM) | $R^1$ | $R^2$ | D |
|---|---|---|---|---|---|
| 29 | 105-9 | 0,40 (A) | $-OCH_3$ | $-OCH_3$ | $4-C_6H_5$ |
| 30 | 90 | 0,64 A) | $-OCH_3$ | $-OCH_3$ | $4-CF_3$ |
| 31 | 69 | 0,55 (A) | $-OCH_3$ | $-OCH_3$ | $3-CF_3$ |
| 32 | 81,5 | 0,41 (A) | $-CH_3$ | $-OCH_3$ | $3-CF_3$ |
| 33 | 120 | 0,53 (A) | $-OCH_3$ | $-OCH_3$ | 4-Cl, $3-CF_3$ |
| 34 | 96 | 0,41 (A) | $-CH_3$ | $-OCH_3$ | 4-Cl, $3-CF_3$ |
| 35 | 86 | 0,29 (A) | $-CH_3$ | $-CH_3$ | 4-Cl, $3-CF_3$ |
| 36 | 106 | 0,32 (A) | $-CH_3$ | $-CH_3$ | $(3,4,5)-F_3$ |
| 37 | 50 | 0,27 (A) | $-CH_3$ | $-CH_3$ | $4-CF_3$ |
| 38 | 91 | 0,31 (A) | $-CH_3$ | $-CH_3$ | $3-CF_3$ |

**Tabelle 2:**

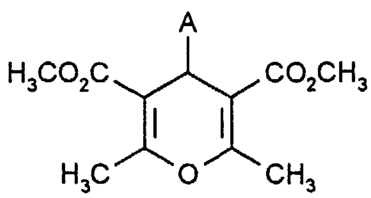

| Bsp.-Nr. | A | F (°C) | $R_f$-Wert (LM) |
|---|---|---|---|
| 39 | 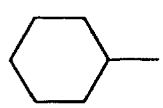 | 68-70 | 0.49(A) |
| 40 | α-Naphthyl | 127-9 | 0,40 (A) |

$R_f$-Wert LM: A: Toluol/Essigsäureethylester = 10:1

B: " = 3:1

**Beispiel 41**

5-Acetyl-4-(4-chlor-3-trifluormethylphenyl)-2,6-dimethyl-4H-pyran-3-carbonsäureamid

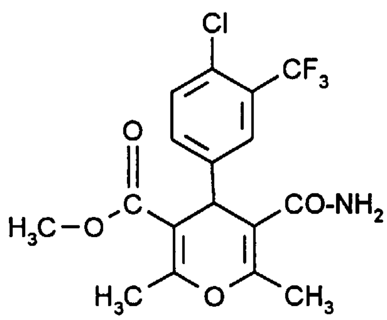

**Vorstufe a)**

1,01 g (2,57 mmol) der Verbindung aus Beispiel 5 werden in 10 ml THF gelöst und mit 10 ml MeOH sowie 10 ml 1N NaOH versetzt und über Nacht bei RT gerührt. Dann wird das Lösemittel abdestilliert und der Rückstand zwischen Wasser und $Et_2O$ verteilt. Die wäßrige Phase wird auf pH = 5 gestellt und der entstehende Niederschlag abgesaugt und gewaschen. Man erhält 432 mg (45 % d.Th.) eines farblosen Pulvers, das für weitere Umsetzungen genügende Reinheit aufweist.

**Vorstufe b)**

1 g der obigen Säure wird in 10 ml $SOCl_2$ gelöst und 1,5 h am Rückfluß gehalten. Dann wird das Reagenz abdestilliert und der Rückstand in 50 ml THF aufgenommen.

Man tropft unter Kühlung 10 ml 25 %iges Ammoniakwasser zu und rührt 30 min bei RT nach. Dann wird eingeengt und der Rückstand zwischen Essigsäureethylester (AcOEt) und $H_2O$ verteilt. Nach Extraktion (AcOEt) wird getrocknet ($MgSO_4$) und eingeengt.

Das Rohprodukt wird durch Chromatographie an Kieselgel (Petrolether/AcOH, Gradient) gereinigt. Man erhält 319 mg, 31 % eines hellbraunen Pulvers.

Schmelzpunkt: 129°C

$R_f$= 0,65

NMR: ($CDCl_3$ 200 MHz): 2,18 (s, 3H); 2,24 (s, 3H); 2,35 (s, 3H); 4,82 (s, 1H); 5,10- 5,40 (m, br, 2H) und 7,31 - 7,52 ppm (m, 3H).

**Beispiele 42 und 43**

4-(4-Chlorphenyl)-2,6-dimethyl-4H-pyran-3,5-dicarbonsäure-3-methylester-5-N-methylamid-und 4-(4-Chlorphenyl)-2,6-dimethyl-4H-pyran-3,5-dicarbonsäure-di-N-methylamid

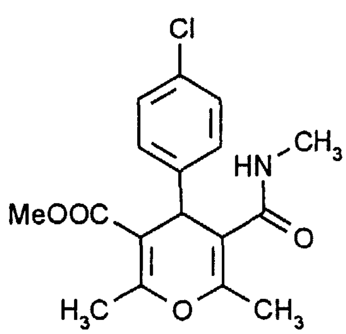

(42)

und

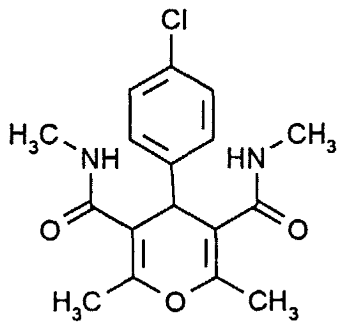

(43)

Zu der Suspension von 1,35 g (20 mmol) Methylammoniumchlorid in 20 ml abs. Toluol werden bei 5°C unter Argon langsam 2,0 ml Trimethylaluminium-Lösung (5 m in n-Hexan) eingetragen. Man läßt die Temperatur auf 25°C ansteigen, rührt 1 bis 2 Stunden nach, bis die Gasentwicklung beendet ist, und erhält eine klare Lösung (1 m Lösung des Reagenz).

In die so erhaltene Lösung des Reagenz (20 mmol) werden 2,0 g (6 mmol) 4-(4-Chlorphenyl)-2,6-dimethyl-4H-pyran-3,5-dicarbonsäuredimethylester in 60 ml abs, Toluol unter Argon bei 25°C getropft. Nach 12 stündigem Erhitzen auf 80°C (DSC-Kontrolle des Umsatzes) wird das Gemisch auf 25°C gekühlt und vorsichtig mit 5 %iger wäßriger Salzsäure angesäuert. Die organische Phase wird abgetrennt und die wäßrige Phase 3 mal mit Essigsäureethylester extrahiert. Die vereinigten organischen Phasen werden mit Wasser bis zur Neutralisation gewaschen, über Natriumsulfat getrocknet, filtriert und i.Vak. eingeengt. Das Rohprodukt (1,5 g) wird an Kieselgel mit Toluol/Essigsäureethylester/Methanol (Gradient) getrennt und liefert 0,1 g Edukt, 0,7 g Mono-N-methylamid sowie 0,7 g Bis-N-Methylamid.

4-(4-Chlorphenyl)-2,6-dimethyl-4H-pyran-3,5-dicarbonsäure-3-methylester-5-N-methylamid (Beispiel 42)

Schmelzpunkt: 163-165°C (Kap.) (aus Dichlormethan/Petrolether), $R_f$-Wert (Toluol/Essigsäureethylester 3:1): 0,18.

| | | | | |
|---|---|---|---|---|
| $C_{17}H_{18}Cl\ NO_4$ | Ber. | C 60,81 % | H 5,40 % | N 4,17 % |
| (335,8) | Gef. | 60,6 % | 5,36 % | 4,23 % |

NMR ($CDCl_3$): 2,20 (s, 3 H), 2,30 (s, 3 H), 2,70 (d, 6 H), 3,60 (s, 3 H), 4,55 (s, 1 H), 5,20 (br, s, 1 H) und 7,20-7,30 ppm (m, 4 H),

4-(4-Chlorphenyl)-2,6-dimethyl-4H-pyran-3,5-dicarbonsäure-di-N-methylamid (Beispiel 43)

Schmelzpunkt: 256-259°C (Kap.) (aus Essigsäureethylester), $R_f$-Wert (Essigsäureethylester): 0,13

| | | | | |
|---|---|---|---|---|
| $C_{17}H_{18}Cl\ NO_4$ | Ber. | C 60,9 % | H 5,70 % | N 8,66 % |
| (334,8) | Gef. | 60,99 % | 5,72 % | 8,37 % |

NMR ($CDCl_3$): 2,13 (s, 6 H), 2,70 (d, 6 H), 4.50 (s, 1 H), 5,30 (br, s, 2 H), und 7,20 - 7,35 ppm (m, 4 H).

In Analogie zu den oben aufgeführten Vorschriften der Beispiele 41 bis 43 werden die in der Tabelle genannten Beispiele hergestellt.

## Tabelle 3

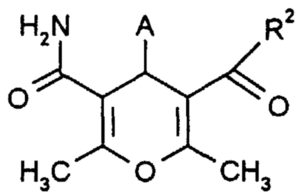

| Bsp.-Nr. | A | $R^2$ | Schmp. | $R_f$ |
|---|---|---|---|---|
| 44<br>45 | 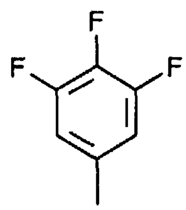 | $NH_2$<br>$OCH_3$ | >220°<br>133° | 0,33 (AcOEt)<br>0,74 (AcOEt) |
| 46<br>41 | 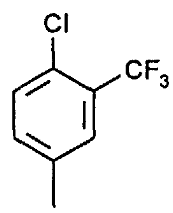 | $NH_2$<br>$OCH_3$ | >220°<br>154° | 0,35 (AcOEt)<br>0,89 (AcOEt) |
| 47<br>48 | 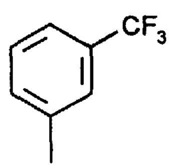 | $NH_2$<br>$OCH_3$ | 210°C<br>142,5 | 0,55 (AcOEt)<br>0,94 (AcOEt) |
| 49<br>50 | 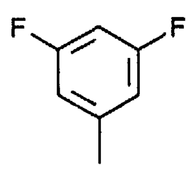 | $NH_2$<br>$OCH_3$ | >220°<br>167,5° | 0,39 (AcOEt)<br>0,8 (AcOEt) |

## Patentansprüche

**1.** Substituierte 4H-Pyrane der allgemeinen Formel

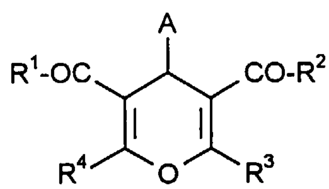

(I)

in welcher

A für Cycloalkyl mit 3 bis 6 Kohlenstoffatomen oder für geradkettiges oder verzweigtes Alkyl mit bis zu 8 Kohlenstoffatomen steht, oder für Aryl mit 6 bis 10 Kohlenstoffatomen oder für Pyridyl steht, die gegebenenfalls bis zu 3-fach gleich oder verschieden durch Nitro, Hydroxy, Carboxyl, Cyano, Aryl mit 6 bis 10 Kohlenstoffatomen, Halogen, Cycloalkyl mit 3 bis 6 Kohlenstoffatomen, Trifluormethyl oder durch geradkettiges oder verzweigtes Alkyl, Alkoxycarbonyl, Alkylthio oder Alkoxy mit jeweils bis zu 6 Kohlenstoffatomen oder durch eine Gruppe der Formel -O-CO-$R^5$ substituiert sind,
worin

$R^5$ geradkettiges oder verzweigtes Alkyl mit bis zu 6 Kohlenstoffatomen bedeutet,

$R^1$ und $R^2$ gleich oder verschieden sind und für Wasserstoff, Amino oder für geradkettiges oder verzweigtes Alkyl, Alkoxy oder Alkylamino mit jeweils bis zu 8 Kohlenstoffatomen stehen,

$R^3$ und $R^4$ gleich oder verschieden sind und für geradkettiges oder verzweigtes Alkyl oder Acyl mit jeweils bis zu 6 Kohlenstoffatomen stehen,

und deren Salze,
wobei 3,5-Diacetyl-2,4,6-trimethyl-4H-Pyran, 3,5-Diethoxycarbonyl-2,6-dimethyl-4-phenyl-4H-Pyran und 3,5-Diethoxycarbonyl-2,4,6-trimethyl-4H-Pyran ausgenommen sind.

**2.** Substituierte 4H-Pyrane der Formel nach Anspruch 1
in welcher

A für Cyclopropyl, Cyclobutyl, Cyclopentyl oder Cyclohexyl steht, oder für geradkettiges oder verzweigtes Alkyl mit bis zu 6 Kohlenstoffatomen steht, oder für Phenyl, Naphthyl oder Pyridyl steht, die gegebenenfalls bis zu 3-fach gleich oder verschieden durch Nitro, Cyano, Fluor, Chlor, Brom, Iod, Phenyl, Naphthyl, Trifluormethyl, Hydroxy, Carboxyl, Cyclopropyl, Cyclopentyl, Cyclohexyl oder durch geradkettiges oder verzweigtes Alkyl, Alkoxycarbonyl, Alkylthio oder Alkoxy mit jeweils bis zu 4 Kohlenstoffatomen oder durch eine Gruppe der Formel -O-CO-$R^5$ substituiert sind,
worin

$R^5$ geradkettiges oder verzweigtes Alkyl mit bis zu 4 Kohlenstoffatomen bedeutet,

$R^1$ und $R^2$ gleich oder verschieden sind und für Wasserstoff, Amino oder für geradkettiges oder verzweigtes Alkyl, Alkoxy oder Alkylamino mit jeweils bis zu 6 Kohlenstoffatomen stehen,

$R^3$ und $R^4$ gleich oder verschieden sind und für geradkettiges oder verzweigtes Alkyl oder Acyl mit jeweils bis zu 4 Kohlenstoffatomen stehen,

und deren Salze,
wobei 3,5-Diacetyl-2,4,6-trimethyl-4H-Pyran, 3,5-Diethoxycarbonyl-2,6-dimethyl-4-phenyl-4H-Pyran und 3,5-Diethoxycarbonyl-2,4,6-trimethyl-4H-Pyran ausgenommen sind.

**3.** Substituierte 4H-Pyrane der Formel nach Anspruch 1 in welcher

A für Cyclopropyl, Cyclohexyl oder für geradkettiges oder verzweigtes Alkyl mit bis zu 4 Kohlenstoffatomen steht, oder für Naphthyl oder Phenyl steht, die gegebenenfalls bis zu 3-fach gleich oder verschieden durch Nitro, Cyano, Fluor, Chlor, Brom, Iod, Phenyl, Naphthyl, Hydroxy, Carboxyl, Cyclopropyl, Cyclopentyl, Cyclohexyl, Trifluormethyl oder durch geradkettiges oder verzweigtes Alkyl, Alkoxycarbonyl, Alkylthio oder Alkoxy mit jeweils bis zu 4 Kohlenstoffatomen oder durch eine Gruppe der Formel -O-CO-$R^5$ substituiert sind, worin

$R^5$ geradkettiges oder verzweigtes Alkyl mit bis zu 4 Kohlenstoffatomen bedeutet,

$R^1$ und $R^2$ gleich oder verschieden sind und für Wasserstoff, Amino oder für geradkettiges oder verzweigtes Alkyl, Alkoxy oder Alkylamino mit jeweils bis zu 4 Kohlenstoffatomen stehen,

$R^3$ und $R^4$ gleich oder verschieden sind und für geradkettiges oder verzweigtes Alkyl oder Acyl mit jeweils bis zu 4 Kohlenstoffatomen stehen,

und deren Salze, wobei 3,5-Diacetyl-2,4,6-trimethyl-4H-pyran, 3,5-Diethoxycarbonyl-2,6-dimethyl-4-phenyl-4H-pyran und 3,5-Diethoxycarbonyl-2,4,6-trimethyl-4H-pyran ausgenommen sind.

**4.** Substituierte 4H-Pyrane nach Anspruch 1 bis 3 zur Verwendung als Arzneimittel.

**5.** 3,5-Diacetyl-2,4,6-trimethyl-4H-pyran, 3,5-Diethoxycarbonyl-2,6-dimethyl-4-phenyl-4H-pyran und 3,5-Diethoxycarbonyl-2,4,6-trimethyl-4H-pyran zur Verwendung als Arzneimittel.

**6.** Verfahren zur Herstellung von substituierten 4H-Pyranen nach Anspruch 1 bis 3, dadurch gekennzeichnet, daß man

[A] Aldehyde der allgemeinen Formel (II)

A-CHO (II)

in welcher
A die oben angegebene Bedeutung hat,
mit Verbindungen der allgemeinen Formel (III)

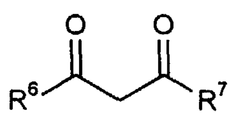

(III)

in welcher

$R^6$ die oben angegebenen Bedeutungen von $R^1$ und $R^2$ umfaßt,

und

$R^7$ die oben angegebenen Bedeutungen von $R^3$ und $R^4$ umfaßt,

oder

[B] Ylidenverbindungen der allgemeinen Formel (IV)

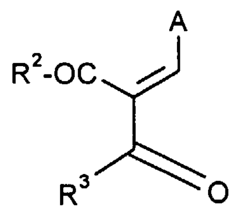

(IV)

in welcher

A, $R^2$ und $R^3$ die oben angegebenen Bedeutungen haben,

mit Verbindungen der allgemeinen Formel (IIIa) oder (IIIb)

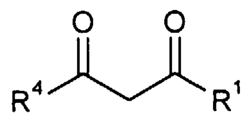

(IIIa)

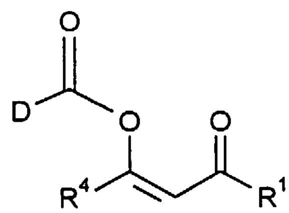

(IIIb)

in welchen

$R^1$ und $R^4$ die oben angegebenen Bedeutungen haben und

D zusammen mit der CO-Gruppe einen elektronenziehenden, aktivierten Rest bildet, wobei D beispielsweise für Wasserstoff, Trifluormethyl, Phenyl oder für $C_1$-$C_4$-Alkyl, vorzugs-weise für Methyl steht,

in inerten Lösemitteln, in Anwesenheit von Hilfsstoffen und/oder in Anwesenheit eines wasserentziehenden Mittels umsetzt
und im Fall der Verbindungen der allgemeinen Formel (I), in welcher $R^1$/$R^2$ für Amino und/oder Alkylamino stehen,
ausgehend von den Estern zunächst durch Verseifung die entsprechenden Säuren herstellt, in die Carbonsäurechloride überführt und in einem letzten Schritt mit Ammoniak oder Alkylaminen umsetzt.

**7.** Arzneimittel enthaltend mindestens ein substituiertes 4H-Pyran nach Anspruch 1 bis 3.

**8.** Arzneimittel nach Anspruch 7 zur Behandlung von Erkrankungen des zentralen Nervensystems.

**9.** Verwendung von 4H-Pyranen nach Anspruch 1 bis 3 zur Herstellung von Arzneimitteln.

**10.** Verwendung von 4H-Pyranen nach Anspruch 1 bis 3 zur Herstellung von Arzneimitteln zur Behandlung von zentral degenerativen Erkrankungen.

**11.** Verwendung von 3,5-Diacetyl-2,4,6-trimethyl-4H-pyran, 3,5-Diethoxycarbonyl-2,6-dimethyl-4-phenyl-4H-pyran und 3,5-Diethoxycarbonyl-2,4,6-trimethyl-4H-pyran zur Herstellung von Arzneimitteln.

**12.** Verwendung von 3,5-Diacetyl-2,4,6-trimethyl-4H-pyran, 3,5-Diethoxycarbonyl-2,6-dimethyl-4-phenyl-4H-pyran und 3,5-Diethoxycarbonyl-2,4,6-trimethyl-4H-pyran zur Herstellung von Arzneimitteln zur Behandlung von zentral degenerativen Erkrankungen.

## Claims

**1.** Substituted 4H-pyrans of the general formula

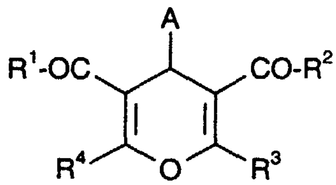

(I)

in which

A represents cycloalkyl having 3 to 6 carbon atoms or represents straight-chain or branched alkyl having up to 8 carbon atoms, or represents aryl having 6 to 10 carbon atoms or represents pyridyl, which are optionally substituted up to 3 times by identical or different substituents consisting of nitro, hydroxyl, carboxyl, cyano, aryl having 6 to 10 carbon atoms, halogen, cycloalkyl having 3 to 6 carbon atoms, trifluoromethyl or by straight-chain or branched alkyl, alkoxycarbonyl, alkylthio or alkoxy having in each case up to 6 carbon atoms, or by a group of the formula -O-CO-$R^5$,
in which

$R^5$ denotes straight-chain or branched alkyl having up to 6 carbon atoms,

$R^1$ and $R^2$ are identical or different and represent hydrogen, amino or represent straight-chain or branched alkyl, alkoxy or alkylamino having in each case up to 8 carbon atoms,

$R^3$ and $R^4$ are identical or different and represent straight-chain or branched alkyl or acyl having in each case up to 6 carbon atoms,

and salts thereof,
with the exception of 3,5-diacetyl-2,4,6-trimethyl-4H-pyran, 3,5-diethoxycarbonyl-2,6-dimethyl-4-phenyl-4H-pyran and 3,5-diethoxycarbonyl-2,4,6-trimethyl-4H-pyran.

**2.** Substituted 4H-pyrans of the formula (I) according to Claim 1
in which

A represents cyclopropyl, cyclobutyl, cyclopentyl or cyclohexyl, or represents straight-chain or branched alkyl having up to 6 carbon atoms, or represents phenyl, naphthyl or pyridyl which are optionally substituted up to 3 times by identical or different substituents consisting of nitro, cyano, fluoro, chloro, bromo, iodo, phenyl, naphthyl, trifluoromethyl, hydroxyl, carboxyl, cyclopropyl, cyclopentyl, cyclohexyl or by straight-chain or branched alkyl, alkoxy carbonyl, alkylthio or alkoxy having in each case up to 4 carbon atoms, or by a group of the formula -O-CO-$R^5$
in which

$R^5$ denotes straight-chain or branched alkyl having up to 4 carbon atoms,

$R^1$ and $R^2$ are identical or different and represent hydrogen, amino or represent straight-chain or branched alkyl, alkoxy or alkylamino having in each case up to 6 carbon atoms,

$R^3$ and $R^4$ are identical or different and represent straight-chain or branched alkyl or acyl having in each case up to 4 carbon atoms,

and salts thereof,
with the exception of 3,5-diacetyl-2,4,6-trimethyl-4H-pyran, 3,5-diethoxycarbonyl-2,6-dimethyl-4-phenyl-4H-pyran

and 3,5-diethoxycarbonyl-2,4,6-trimethyl-4H-pyran.

**3.** Substituted 4H-pyrans of the formula (I) according to Claim 1
in which

A represents cyclopropyl, cyclohexyl or represents straight-chain or branched alkyl having up to 4 carbon atoms, or
represents naphthyl or phenyl which are optionally substituted up to 3 times by identical or different substituents consisting of nitro, cyano, fluoro, chloro, bromo, iodo, phenyl, naphthyl, hydroxyl, carboxyl, cyclopropyl, cyclopentyl, cyclohexyl, trifluoromethyl, or by straight-chain or branched alkyl, alkoxycarbonyl, alkylthio or alkoxy having in each case up to 4 carbon atoms, or by a group of the formula -O-CO- $R^5$
in which

$R^5$ denotes straight-chain or branched alkyl having up to 4 carbon atoms,

$R^1$ and $R^2$ are identical or different and represent hydrogen, amino or represent straight-chain or branched alkyl, alkoxy or alkylamino having in each case up to 4 carbon atoms,

$R^3$ and $R^4$ are identical or different and represent straight-chain or branched alkyl or acyl having in each case up to 4 carbon atoms,

and salts thereof,
with the exception of 3,5-diacetyl-2,4,6-trimethyl-4H-pyran, 3,5-diethoxycarbonyl-2,6-dimethyl-4-phenyl-4H-pyran and 3,5-diethoxycarbonyl-2,4,6-trimethyl-4H-pyran.

**4.** Substituted 4H-pyrans according to Claim 1 to 3 for use as medicaments.

**5.** 3,5-Diacetyl-2,4,6-trimethyl-4H-pyran, 3,5-diethoxycarbonyl-2,6-dimethyl-4-phenyl-4H-pyran and 3,5-diethoxycarbonyl-2,4,6-trimethyl-4H-pyran for use as medicaments.

**6.** Process for the preparation of substituted 4H-pyrans according to Claim 1 to 3, characterized in that

[A] aldehydes of the general formula (II)

A-CHO (II)

in which
A has the meaning given above
are reacted with compounds of the general formula (III)

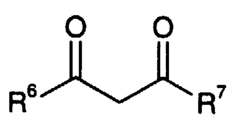

(III)

in which

$R^6$ embraces the meanings of $R^1$ and $R^2$ given above in each case

and

$R^7$ embraces the meanings of $R^3$ and $R^4$ given above,

or

[B] ylidene compounds of the general formula (IV)

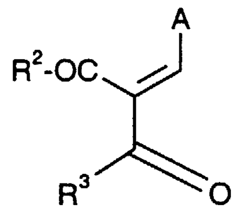

(IV)

in which

A, $R^2$ and $R^3$ have the meanings given above,

are reacted with compounds of the general formula (IIIa) or (IIIb)

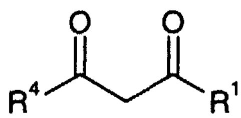

(IIIa)

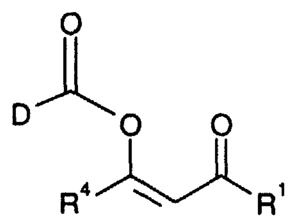

(IIIb)

in which

$R^1$ and $R^4$ have the meanings given above and

D together with the CO group, forms an electron-attracting, activated radical, with D representing for example hydrogen, trifluoromethyl, phenyl or $C_1$-$C_4$-alkyl, preferably methyl,

in inert solvents in the presence of auxiliaries and/or in the presence of a dehydrating agent, and, in the case of the compounds of the general formula (I) in which $R^1$/$R^2$ represent amino and/or alkylamino, the corresponding acids are first of all prepared by hydrolysis from the esters, are converted into the carbonyl chlorides by transesterification, for example with thionyl chloride, and in a final step are reacted with ammonia or alkylamines.

**7.** Medicaments comprising at least one substituted 4H-pyran according to Claim 1 to 3.

**8.** Medicaments according to Claim 7 for treating disorders of the central nervous system.

**9.** Use of 4H-pyrans according to Claim 1 to 3 for the production of medicaments.

**10.** Use of 4H-pyrans according to Claim 1 to 3 for the production of medicaments for the treatment of degenerative CNS disorders.

**11.** Use of 3,5-diacetyl-2,4,6-trimethyl-4H-pyran, 3,5-diethoxycarbonyl-2,6-dimethyl-4-phenyl-4H-pyran and 3,5-diethoxycarbonyl-2,4,6-trimethyl-4H-pyran for the production of medicaments.

**12.** Use of 3,5-diacetyl-2,4,6-trimethyl-4H-pyran, 3,5-diethoxycarbonyl-2,6-dimethyl-4-phenyl-4H-pyran and 3,5-diethoxycarbonyl-2,4,6-trimethyl-4H-pyran for the production of medicaments for the treatment of degenerative CNS disorders.

## Revendications

**1.** 4H-pyrannes substitués de formule générale

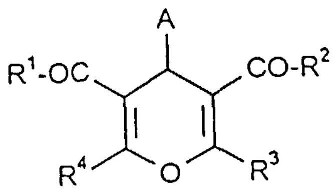

(I)

dans laquelle

A est un groupe cycloalkyle ayant 3 à 6 atomes de carbone, ou bien un groupe alkyle linéaire ou ramifié ayant jusqu'à 8 atomes de carbone, ou bien un groupe aryle ayant 6 à 10 atomes de carbone ou un groupe pyridyle, qui sont substitués éventuellement jusqu'à 3 fois identiques ou différentes par un radical nitro, hydroxy, carboxyle, cyano, aryle ayant 6 à 10 atomes de carbone, halogéno, cycloalkyle ayant 3 à 6 atomes de carbone, trifluorométhyle ou par un groupe alkyle linéaire ou ramifié, alkoxy-carbonyle, alkylthio ou alkoxy ayant chacun jusqu'à 6 atomes de carbone ou par un groupe de formule $-O-CO-R^5$, dans laquelle

$R^5$ est un groupe alkyle linéaire ou ramifié ayant jusqu'à 6 atomes de carbone,

$R^1$ et $R^2$ sont identiques ou différents et représentent l'hydrogène, un groupe amino ou un groupe, linéaire ou ramifié, alkyle, alkoxy ou alkylamino ayant chacun jusqu'à 8 atomes de carbone,

$R^3$ et $R^4$ sont identiques ou différents et représentent un groupe alkyle ou acyle linéaire ou ramifié ayant chacun jusqu'à 6 atomes de carbone,

et leurs sels,

le 3,5-diacétyl-2,4,6-triméthyl-4H-pyranne, le 3,5-diéthoxycarbonyl-2,6-diméthyl-4-phényl-4H-pyranne et le 3,5-diéthoxycarbonyl-2,4,6-triméthyl-4H-pyranne étant exclus.

**2.** 4H-pyrannes substitués de formule suivant la revendication 1 dans laquelle

A est un groupe cyclopropyle, cyclobutyle, cyclopentyle ou cyclohexyle, ou bien un groupe alkyle linéaire ou ramifié ayant jusqu'à 6 atomes de carbone, ou bien un groupe phényle, un groupe naphtyle ou un groupe pyridyle qui sont substitués, le cas échéant jusqu'à 3 fois identiques ou différentes par un radical nitro, cyano, fluoro, chloro, bromo, iodo, phényle, naphtyle, trifluorométhyle, hydroxy, carboxyle, cyclopropyle, cyclopentyle, cyclohexyle ou par un groupe alkyle, alkoxycarbonyle, alkylthio ou alkoxy linéaire ou ramifié ayant chacun jusqu'à 4 atomes de carbone ou par un groupe de formule $-O-CO-R^5$, où

$R^5$ est un groupe alkyle linéaire ou ramifié ayant jusqu'à 4 atomes de carbone,

$R^1$ et $R^2$ sont identiques ou différents et représentent l'hydrogène, un groupe amino ou un groupe alkyle, alkoxy ou alkylamino linéaire ou ramifié ayant chacun jusqu'à 6 atomes de carbone,

$R^3$ et $R^4$ sont identiques ou différents et représentent un groupe alkyle ou acyle linéaire ou ramifié ayant chacun jusqu'à 4 atomes de carbone,

et leurs sels,

le 3,5-diacétyl-2,4,6-triméthyl-4H-pyranne, le 3,5-diéthoxycarbonyl-2,6-diméthyl-4-phényl-4H-pyranne et le 3,5-diéthoxycarbonyl-2,4,6-triméthyl-4H-pyranne étant exclus.

**3.** 4H-pyrannes substitués de formule suivant la revendication 1 dans laquelle

A est un groupe cyclopropyle, cyclohexyle ou un groupe alkyle linéaire ou ramifié ayant jusqu'à 4 atomes de carbone, ou bien
un groupe naphtyle ou un groupe phényle qui sont substitués, le cas échéant jusqu'à 3 fois identiques ou différentes par un radical nitro, cyano, fluoro, chloro, bromo, iodo, phényle, naphtyle, hydroxy, carboxyle, cyclopropyle, cyclopentyle, cyclohexyle, trifluorométhyle ou par un groupe alkyle, alkoxycarbonyle, alkylthio ou alkoxy linéaire ou ramifié ayant chacun jusqu'à 4 atomes de carbone ou par un groupe de formule -O-CO-$R^5$,
dans laquelle

$R^5$ est un groupe alkyle linéaire ou ramifié ayant jusqu'à 4 atomes de carbone,

$R^1$ et $R^2$ sont identiques ou différents et représentent l'hydrogène, un groupe amino ou un groupe alkyle, alkoxy ou alkylamino linéaire ou ramifié ayant chacun jusqu'à 4 atomes de carbone,
$R^3$ et $R^4$ sont identiques ou différents et représentent un groupe alkyle ou acyle linéaire ou ramifié ayant chacun jusqu'à 4 atomes de carbone,

et leurs sels,
le 3,5-diacétyl-2,4,6-triméthyl-4H-pyranne, le 3,5-diéthoxycarbonyl-2,6-diméthyl-4-phényl-4H-pyranne et le 3,5-diéthoxycarbonyl-2,4,6-triméthyl-4H-pyranne étant exclus.

**4.** 4H-pyrannes substitués suivant les revendications 1 à 3, destinés à être utilisés comme médicaments.

**5.** Le 3,5-diacétyl-2,4,6-triméthyl-4H-pyranne, le 3,5-diéthoxycarbonyl-2,6-diméthyl-4-phényl-4H-pyranne et le 3,5-diéthoxycarbonyl-2,4,6-triméthyl-4H-pyranne destinés à être utilisés comme médicaments.

**6.** Procédé de production de 4H-pyrannes substitués suivant les revendications 1 à 3, caractérisé en ce que

[A] on fait réagir des aldéhydes de formule générale (II)

A-CHO (II)

dans laquelle

A a la définition indiquée ci-dessus,

avec des composés de formule générale (III)

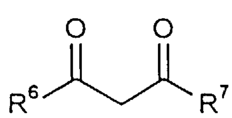
(III)

dans laquelle

$R^6$ a les définitions indiquées ci-dessus pour $R^1$ et $R^2$,

et

$R^7$ a les définitions indiquées ci-dessus pour $R^3$ et $R^4$,

ou bien
[B] des composés ylidéniques de formule générale (IV)

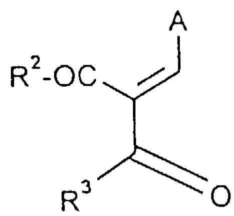

(IV)

dans laquelle

A, $R^2$ et $R^3$ ont les définitions indiquées ci-dessus,

avec des composés de formule générale (IIIa) ou (IIIb)

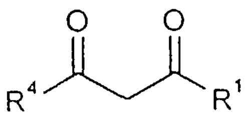

(IIIa)

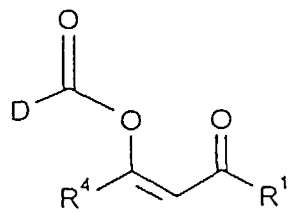

(IIIb)

formules dans lesquelles

$R^1$ et $R^4$ ont les définitions indiquées ci-dessus et
D forme conjointement avec le groupe CO un reste activé attirant les électrons, D représentant, par exemple l'hydrogène, le groupe trifluorométhyle, phényle ou un groupe alkyle en $C_1$ à $C_4$, de préférence le groupe méthyle,

dans des solvants inertes en présence de substances auxiliaires et/ou en présence d'un agent déshydratant et

dans le cas des composés de formule générale (I), dans laquelle $R^1/R^2$ représentent un groupe amino et/ou alkylamino,

on prépare tout d'abord à partir des esters par saponification les acides correspondants, on les transforme en chlorures d'acides carboxyliques et on les fait réagir dans une dernière étape avec l'ammoniac ou des alkylamines.

**7.** Médicament contenant au moins un 4H-pyranne substitué suivant les revendications 1 à 3.

**8.** Médicament suivant la revendication 7, destiné au traitement de maladies du système nerveux central.

**9.** Utilisation de 4H-pyrannes suivant les revendications 1 à 3 pour la préparation de médicaments.

**10.** Utilisation de 4H-pyrannes suivant les revendications 1 à 3 pour la préparation de médicaments destinés au traitement de maladies dégénératives du système nerveux central.

**11.** Utilisation du 3,5-diacétyl-2,4,6-triméthyl-4H-pyranne, du 3,5-diéthoxycarbonyl-2,6-diméthyl-4-phényl-4H-pyranne et du 3,5-diéthoxycarbonyl-2,4,6-triméthyl-4H-pyranne pour la préparation de médicaments.

**12.** Utilisation du 3,5-diacétyl-2,4,6-triméthyl-4H-pyranne, du 3,5-diéthoxycarbonyl-2,6-diméthyl-4-phényl-4H-pyranne et du 3,5-diéthoxycarbonyl-2,4,6-triméthyl-4H-pyranne pour la préparation de médicaments destinés au traitement de maladies dégénératives du système nerveux central.